# EUROPEAN PATENT APPLICATION

(11) **EP 4 488 388 A1**
(43) Date of publication of application: **08.01.2025**
(21) Application number: 23199496.3
(22) Date of filing: 25.09.2023
(51) Int. Cl.: C12Q 1/6883

(54) **SYSTEMS AND METHODS FOR PREDICTING RESPONSE TO ORAL MINOXIDIL FOR THE TREATMENT OF ANDROGENETIC ALOPECIA**

(30) Priority: 06.07.2023 US 202363512137 P; 31.08.2023 US 202318240485
(71) Applicant: Follea International, Irvine California 92614 (US)
(72) Inventor: Goren, Ofer Andy, 11000 Prague (CZ)
(74) Representative: Schaafhausen Patentanwälte PartGmbB

(57) **Abstract**

Methods, processes, systems, and apparatuses are disclosed for predicting minoxidil response in the treatment of androgenetic alopecia, and prescribing a minoxidil dosage amount, minoxidil dosage frequency, and/or minoxidil dosage routine based on the same.

## Description

### TECHNICAL FIELD

The inventions described here relate to systems and methods for predicting response to oral minoxidil for the treatment of androgenetic alopecia.

### BACKGROUND

Hair loss is associated with a variety of psychological and social implications. Prior to starting any treatment it is advantageous to predict the course, severity, and treatment options of the disease. In the field of hair loss, very little scientific diagnostic tests are currently available, and there are few methods to predict treatment response.

Moreover, the hair loss industry is littered with dozens of products that claim to grow, improve, and replace hair. Unfortunately, few treatments have been scientifically demonstrated to work, and the few treatments that have undergone clinical trials often do not work equally for all patients.

Androgenetic alopecia has been successfully treated in men by the U.S. Food & Drug Administration ("FDA") approved medications minoxidil (marketed as Rogaine^{™} or Regaine^{™}). Minoxidil has also been approved by the FDA for treatment of female hair loss; however, for most women minoxidil is only marginally successful atretaining existing hair. Some men for whom minoxidil is less effective have been successfully treated with finasteride; however, the same cannot be said of females for whom minoxidil is ineffective. Studies have thus far failed to show the effectiveness of finasteride in the treatment of female androgenetic alopecia.

There are significant differences between male androgenetic alopecia and female androgenetic alopecia. Apart from the different baldness patterns, male and female alopecia follow a different mechanism. In men, alopecia is related to the normal high androgen levels in males, combined with an underlying sensitivity of the hair roots to androgens. Women, however, have roughly 10 times lower androgen levels than men, and the absolute amount of androgen is a less significant factor than the increased sensitivity of the hair roots to androgens.

Among various individuals, whether male or female, there is a broad variability in the response of different people to various hair loss treatments. This variability is presumed to be a result of genetic factors contributing to variable enzyme activity in the follicles, making a one- size-fits-all approach difficult to achieve. It would therefore be advantageous to be able to have an effective diagnostic and treatment method where patients could be selected and treated on the basis of criteria such as enzyme activity, which would identify some people as being likely to benefit from treatment by minoxidil and other drugs, while identifying other individuals in which treatment is not likely to be effective. Previous methods of predicting response to minoxidil are explained in United States Patent No. 8,691,518. However, there still existed a need to predict response to oral forms of minoxidil versus topical forms of minoxidil.

### BRIEF SUMMARY

The inventions described here relate to systems and methods for predicting oral and/or topical form minoxidil response in the treatment of androgenetic alopecia, which in one embodiment may be based on a colorimetric assay. Various embodiments are possible, a number of which are exemplified here. In particular, variations in hair follicle minoxidil sulfotransferase activity may be used to predict the efficacy of minoxidil for the treatment of androgenetic alopecia.

In one embodiment of the present disclosure, there is provided a method for selecting a treatment for a subject suffering from androgenetic alopecia, comprising obtaining a sample of one or more hair follicles, or a scalp biopsy, from the subject; performing an assay to measure minoxidil sulfotransferase activity in the sample, thereby generating an activity value indicative of the minoxidil sulfotransferase activity level in the sample; comparing the activity value to one or more standardized activity values, each standardized activity value representing either high or low expected minoxidil response for hair re-growth or retention for a class of patients including the subject, thereby producing an indication of either high or low expected minoxidil response for hair re-growth or retention for the subject at a particular dosage of minoxidil; and presenting the indication to the subject. Optionally, the indication of high or low expected minoxidil response for hair re-growth or retention is used to select oral or topical minoxidil treatment.

In another embodiment, there is provided a composition of matter for performing an assay comprising about 30 to about 70 mM potassium phosphate buffer (pH 6.5); about 3 to about 7 mM magnesium chloride; about 15 to about 25 µM adenosine 3',5'-diphosphate (PAP) or adenosine 3'-phosphate, 5'-phosphosulfate (PAPS); about 3 to about 7 mMp-nitrophenyl sulfate; and about 0.07 to about 0.13 mM minoxidil.

In another embodiment, there is provided a kit comprising a transparent container with a lid, comprising the above composition for performing an assay; a buffer container comprising about 0.20 M to about 0.30 M Tris-HCl, pH 8.7; and means for mixing the contents of the buffer container with the contents of the apparatus.

As will be explained herein, embodiments can relate to a method for selecting a treatment for a human subject suffering from androgenetic alopecia. The method can involve obtaining a sample from the human subject. The sample can include one or more hairs that have been plucked from the human subject. The one or more hairs can include one or more hair follicles. The method can involve performing a colorimetric assay to measure minoxidil sulfotransferase activity in the sample, thereby generating an activity value indicative of the minoxidil sulfotransferase activity level in the sample. In some embodiments, the step of placing the sample in a reaction mixture can include use of an indicator dye and minoxidil. During the assay, the indicator dye can undergoes a color change that correlates with the amount of minoxidil sulfotransferase activity level in the sample. The activity value can correlates with the color change. The method can involve comparing the activity value to one or more standardized activity values. Each standardized activity value can represent either high or low expected minoxidil response for hair re-growth or retention for a class of patients including the subject, thereby producing an indication of either high or low expected minoxidil response for hair re-growth or retention for the subject at a particular dosage of minoxidil. The method can involve presenting the indication to the human subject.

In some embodiments, the indication can be of high expected minoxidil response for hair re-growth or retention, and the particular dosage of minoxidil can be selected from the group consisting of approximately 2% topical solution, approximately 5% topical solution, approximately 2% topical foam, approximately 5% topical foam, approximately 0.625 mg, 1.25 mg, 2.5 mg tablet, and approximately 5 mg tablet.

In some embodiments, the assay can include the steps of: placing the sample in a reaction mixture containing about 50 mM potassium phosphate buffer with pH between about 6.5 and about 8.0, about 5 mM magnesium chloride, about 20 µM adenosine 3',5'-diphosphate (PAP) or adenosine 3'-phosphate, 5'-phosphosulfate (PAPS), about 5 mM p-nitrophenyl sulfate and about 0.1 mM minoxidil; mixing the reaction mixture which contains the sample, such that a reaction is initiated; incubating the sample within the reaction mixture for a predetermined length; and stopping the reaction.

In some embodiments, the potassium phosphate buffer can have a pH of about 8.0.

In some embodiments, a basic buffer of about 0.25 M Tris-HCl, pH approximately 8.7 can be added to the reaction mixture to quench the assay and improve sensitivity of the assay.

In some embodiments, the reaction mixture can be incubated for about 30 minutes to about 24 hours at about 37° C.

In some embodiments, the sample within the reaction mixture can be incubated at room temperature.

In some embodiments, the reaction mixture can be incubated above room temperature to increase dye turnover and the signal strength of the colorimetric assay.

In some embodiments, the reaction mixture can be packaged in a capsule and dissolved in a solution along with the sample.

In some embodiments, the one or more standardized values can be one or more minoxidil sulfotransferase activity levels at which a statistical sample of other subjects show either high or low minoxidil response for hair re-growth or retention at the particular dosage of minoxidil.

In some embodiments, the step of presenting can include sending an electronic signal.

In some embodiments, the indicator dye can be p-nitrophenyl sulfate.

In some embodiments, the colorimetric assay can use 2-naphthol as a substrate, to improve the sensitivity or specificity of the assay.

In some embodiments, the sample can consist of one hair comprising one hair follicle that has been plucked from the human subject.

In some embodiments, the reaction time for the assay can be inversely correlated with the number of hair follicles in the reaction.

In some embodiments, the human subject with high minoxidil sulfotransferase activity can be prescribed a low dose of minoxidil and/or a decrease in the frequency of minoxidil application.

In some embodiments, the human subject with low minoxidil sulfotransferase activity can be prescribed a high dose of minoxidil and/or an increase in the frequency of minoxidil application.

In some embodiments, the human subject with low minoxidil sulfotransferase activity can be prescribed a stabilized form of minoxidil sulfate in the form of a cream, solution and/or gel.

In some embodiments, minoxidil sulfate can be stabilized in liposomes or microencapsulation.

In some embodiments, the assay can include a positive (reference) and negative (blank) control to compare to the sample.

In some embodiments, the color change can be visually discernible. In some embodiments, assay can be performed in a transparent container.

In some embodiments, generating an activity value can be performed by comparing the discernible color after the color change to a reference color card.

In some embodiments, the colorimetric assay can be performed in a transparent container, and generating an activity value is performed by measuring the absorbance at approximately 405 nm with a spectrophotometer.

Another exemplary embodiment can relate to a method for predicting response to minoxidil. The method can involve measuring gene, genetic, and/or enzymatic activity in a sample of a patient to generate an activity value indicative of a biochemical activation response to minoxidil. The method can involve comparing the activity value to a standardized activity value. The method can involve predicting a response to minoxidil treatment based on the comparison.

In some embodiments, measuring gene or enzymatic activity can include: measuring SULT1A1 gene expression, a SULT1A1 gene variant expression, and/or SULT1A1 enzymatic activity in the sample; measuring SLC22A9 gene expression, SCL22A9 gene variant expression, SLC22A9 transport activity from a hair cell in the sample, and/or SLC22A9 transporter activity; measuring AABC3 gene expression and/or AABC3 gene variant expression in the sample; and/or measuring blood flow in the patient (e.g., the patient's scalp).

In some embodiments, the SLC22A9 transport activity from a hair cell can be measured using a standard substrate uptake assay with known substrates (e.g., fluorescent substrates, radionuclear substrates, etc.).

In some embodiments, the minoxidil treatment can be oral minoxidil.

In some embodiments, predicting a response to minoxidil treatment can involve:
- when the gene, genetic, and/or enzymatic activity relates to SULT1A1: estimating that the patient will respond to oral minoxidil when the activity value is lower than a threshold standardized activity value, and estimating that the patient will not respond well to oral minoxidil when the activity value is higher than a threshold standardized activity value;
- when the gene, genetic and/or SLC22A9 transporter activity relates to SLC22A9: estimating that the patient will respond to oral minoxidil when the activity value is higher than a threshold standardized activity value, and estimating that the patient will not respond well to oral minoxidil when the activity value is lower than a threshold standardized activity value;
- when the gene and/or genetic activity relates to AABC3: estimating that the patient will respond to oral minoxidil when the activity value is lower than a threshold standardized activity value, and estimating that the patient will not respond well to oral minoxidil when the activity value is higher than a threshold standardized activity value.

In some embodiments, measuring blood flow of the patient (e.g., the patient's scalp) can involve Doppler velocitometry. Predicting a response to minoxidil treatment can involve: estimating that the patient will respond well to oral minoxidil when the Doppler velocity is lower than a threshold value; and estimating that the patient will not respond well to oral minoxidil when the Doppler velocity is higher than the threshold value.

In some embodiments, when the gene, genetic, and/or enzymatic activity relates to SULT1A1: estimating that the patient will respond well to oral minoxidil when the activity value is lower than a threshold standardized activity value that is within a range from 0.37 to 0.4, and estimating that the patient will not respond well to oral minoxidil when the activity value is higher than a threshold standardized activity value that is within a range from 0.37 to 0.4.

In some embodiments, the minoxidil treatment can be treatment for androgenetic alopecia.

In some embodiments, the method can involve generating plural activity values indicative of the biochemical activation response to minoxidil, and comparing the plural activity values to plural standardized activity values.

In some embodiments, the response to minoxidil treatment can be increased or improved hair growth or hair retention.

In some embodiments, predicting a response to minoxidil treatment can involve estimating a biochemical activation response based on a minoxidil dosage amount, a minoxidil dosage frequency, and/or a minoxidil dosage routine.

In some embodiments, the standardized activity value can pertain to biochemical activation response to minoxidil associated with hair growth or hair retention for a class of individuals.

In some embodiments, the standardized activity value can pertain to biochemical activation response to minoxidil associated with hair growth or hair retention at which a statistical sample of subjects show high or low minoxidil response for a minoxidil dosage amount, a minoxidil dosage frequency, and/or a minoxidil dosage routine.

In some embodiments, measuring gene, genetic, and/or enzymatic activity in the sample of a patient can involve obtaining one or more hair follicles and/or one or more scalp biopsies as the sample.

In some embodiments, measuring gene, genetic, and/or enzymatic activity in the sample of a patient can occur before, during, and/or after the patient is being administered a minoxidil treatment.

In some embodiments, measuring gene, genetic, and/or enzymatic activity in the sample of a patient can occur 1 to 14 days prior to the patient being administered a minoxidil treatment.

In some embodiments, measuring gene, genetic, and/or enzymatic activity in the sample of a patient can be performed via an assay device.

In some embodiments, measuring gene, genetic, and/or enzymatic activity in the sample of a patient can be performed via a colorimetric assay device.

In some embodiments, predicting the response to minoxidil treatment can involve implementation of a neural network.

In some embodiments, measuring gene, genetic, and/or enzymatic activity in the sample of the patient can involve obtaining one or more hair follicles and/or one or more scalp biopsies as the sample, and measuring gene, genetic, and/or enzymatic activity in the sample of the patient is performed via an assay device, wherein the method involves: placing the one or more hair follicles or one or more scalp biopsies in a reaction mixture containing potassium phosphate buffer, magnesium chloride, adenosine 3',5'-diphosphate (PAP) or adenosine 3'-phosphate,5'-phosphosulfate (PAPS), p-nitrophenyl sulfate, and minoxidil; mixing the reaction mixture; incubating the reaction mixture for a predetermined length; and stopping the reaction.

Another exemplary embodiment can relate to a method for selecting a treatment for a human subject suffering from androgenetic alopecia. The method can involve measuring gene, genetic and/or enzymatic activity in a sample of a patient to generate an activity value indicative of a biochemical activation response to minoxidil. The method can involve comparing the activity value to a standardized activity value. The method can involve predicting a response to minoxidil treatment based on the comparison. The method can involve prescribing a minoxidil dosage amount, a minoxidil dosage frequency, and/or a minoxidil dosage routine as the androgenetic alopecia treatment.

In some embodiments, measuring gene, genetic, and/or enzymatic activity can include: measuring SULT1A1 gene expression, SULT1A1 gene variant expression, and/or SULT1A1 enzymatic activity in the sample; measuring SLC22A9 gene expression, SCL22A9 gene variant expression, SLC22A9 activity from a hair follicle in the sample, and/or SLC transporter activity; measuring AABC3 gene expression and/or AABC3 gene variant expression in the sample; and/or measuring blood flow in the patient.

In some embodiments, prescribing the minoxidil dosage amount, the minoxidil dosage frequency, and/or the minoxidil dosage routine can involve prescribing oral minoxidil minoxidil.

In some embodiments, measuring blood flow of the patient (e.g., the patient's scalp) can involve Doppler velocitometry. Predicting a response to minoxidil treatment can involve: estimating that the patient will respond well to oral minoxidil when the Doppler velocity is lower than a threshold value; and estimating that the patient will not respond well to oral minoxidil when the Doppler velocity is higher than the threshold value.

In some embodiments, predicting a response to minoxidil treatment can involve:
- when the, gene, genetic and/or enzymatic activity relates to SULT1A1: estimating that the patient will respond to oral minoxidil when the activity value is lower than a threshold standardized activity value, and estimating that the patient will not respond well to oral minoxidil when the activity value is higher than a threshold standardized activity value;
- when the gene, genetic and/or SLC22A9 transporter activity from the hair cell relates to SLC22A9: estimating that the patient will respond to oral minoxidil when the activity value is higher than a threshold standardized activity value, and estimating that the patient will not respond well to oral minoxidil when the activity value is lower than a threshold standardized activity value;
- when the gene and/or genetic activity relates to AABC3: estimating that the patient will respond to oral minoxidil when the activity value is lower than a threshold standardized activity value, and estimating that the patient will not respond well to oral minoxidil when the activity value is higher than a threshold standardized activity value.

In some embodiments, when the gene, genetic, and/or enzymatic activity relates to SULT1A1: estimating that the patient will respond well to oral minoxidil when the activity value is lower than a threshold standardized activity value that is within a range from 0.37 to 0.4, and estimating that the patient will not respond well to oral minoxidil when the activity value is higher than a threshold standardized activity value that is within a range from 0.37 to 0.4.

### BRIEF DESCRIPTION OF THE DRAWINGS

The accompanying drawings, which are incorporated into this specification, illustrate one or more exemplary embodiments of the inventions disclosed herein and, together with the detailed description, serve to explain the principles and exemplary implementations of these inventions. One of skill in the art will understand that the drawings are illustrative only, and that what is depicted therein may be adapted based on the text of the specification or the common knowledge within this field.

In the drawings, where like reference numerals refer to like reference in the specification:
FIG. 1 is a flowchart showing a method of analyzing one or more hair follicles and providing a result.
FIG. 2 shows an example of a computerized system for conducting or analyzing an assay to test hair follicles and providing a result.
FIG. 3 shows a flow diagram of minoxidil sulfonation.

### DETAILED DESCRIPTION

Various example embodiments of the present inventions are described herein in the context of a therapy for androgenetic alopecia.

The description herein is provided in the context of a therapy for androgenetic alopecia. Those of ordinary skill in the art will realize that the following detailed description is illustrative only and is not intended to be in any way limiting. Other embodiments will readily suggest themselves to such skilled persons having the benefit of this disclosure. Reference will now be made in detail to implementations as illustrated in the accompanying drawings. The same reference indicators will be used throughout the drawings and the following detailed description to refer to the same or like parts.

In the interest of clarity, not all of the routine features of the implementations described herein are shown and described. It will, of course, be appreciated that in the development of any such actual implementation, numerous implementation-specific decisions must be made in order to achieve the developer's specific goals, such as compliance with application- and business-related constraints, and that these specific goals will vary from one implementation to another and from one developer to another. Moreover, it will be appreciated that such a development effort might be complex and time-consuming, but would nevertheless be a routine undertaking of engineering for those of ordinary skill in the art having the benefit of this disclosure.

Androgenetic alopecia is extremely common, affecting approximately 60% of men and over 50% of females by the age of 60. Currently, there are two FDA approved medications for the treatment of androgenetic alopecia, finasteride and minoxidil. However, finasteride therapies that are successful at hair re-growth and maintenance in males have failed to show significant improvement in females. Therefore, minoxidil is currently the only FDA approved medication for female hair loss. Further, for minoxidil therapy to be effective it often must be used for a prolonged period of time without knowing if it is effective. One aim of this invention is to screen patients prior to beginning of therapy and/or during therapy to determine which patients will respond best to minoxidil therapy, and to further determine the form (e.g., topical or oral) of minoxidil to which the patients will respond best, thereby providing personalized effective therapy for hair loss.

The invention is based in part on the discovery that minoxidil requires biochemical activation by minoxidil sulfotransferase, or SULT1A1, to form the active minoxidil sulfate metabolite. The exact mechanism of action for minoxidil based treatment of androgenetic alopecia is not completely understood. However, in vitro studies have demonstrated that minoxidil sulfate is the active metabolite of minoxidil. Response to minoxidil for the treatment of androgenetic alopecia has been associated with differences in scalp sulfotransferase activity. Therefore, it is generally accepted that a subject with a high level of minoxidil sulfotransferase activity will generate more minoxidil sulfate, and therefore will likely have a good response to minoxidil for the treatment of androgenetic alopecia. On the other hand, a subject with a low level of minoxidil sulfotransferase activity will not generate much minoxidil sulfate, and will likely have a poor response to minoxidil for the treatment of androgenetic alopecia.

Clinical trials with minoxidil for the treatment of androgenetic alopecia have shown statistically significant results for maintenance and growth of hair. Several studies have demonstrated the level of minoxidil sulfotransferase activity is significantly greater in patients responding to minoxidil for the treatment of androgenetic alopecia. As described herein, the assessment of hair re-growth is based on one or more of the following parameters: patient self assessment, physician assessment using a standardized scale, global photography assessment, hair diameter measurement, average hair length measurement, average hair diameter measurement, and hair weight measurements. The current inventions provide a method of using biochemical variations in minoxidil sulfotransferase activity as a drug response marker for oral and/or topical minoxidil treatment of androgenetic alopecia. Based on the minoxidil sulfotransferase activity level, the method disclosed herein allows a physician or the patient to select the appropriate treatment and dosage thereof for the treatment of androgenetic alopecia.

In accordance with one approach described herein, a patient's hair follicle sample may be obtained. Preferably, at least two hair follicles may be obtained, so that if only one is analyzed, there will be at least one backup if needed. In one embodiment, the hair follicle sample is collected prior to start of minoxidil therapy. In another embodiment, the hair follicle sample is collected after beginning minoxidil therapy.

The patient's hair follicle sample may be subjected to a colorimetric assay to determine the level of minoxidil sulfotransferase activity. A patient's hair follicle sample (from 1 ton hair follicles) may be placed in a reaction mixture containing about 50 mM potassium phosphate buffer (pH approximately 6.5), about 5 mM magnesium chloride, about 20 µM adenosine 3',5'-diphosphate (PAP) or adenosine 3'-phosphate,5'-phosphosulfate (PAPS), about 5 mM p-nitrophenyl sulfate and about 0.1 mM minoxidil.

In one embodiment this reaction may take place in a transparent container with a lid or other opening in which the hair follicle samples may be inserted. In one non-limiting example, the total amount of liquid in the assay container may be about 0.2 ml.

As part of the above reaction, it is understood that in the presence of minoxidil sulfotransferase activity, p-nitrophenyl sulfate is converted to the colorimetric p-nitrophenolate.

The reaction may be mixed and then incubated for approximately 4 to 16 hours at 37 °C depending on the number of hair follicles used in the assay. Mixing may be by any mixing means known in the art, including shaking the container. Where a shorter incubation time is required for a greater number of hair follicles. In one embodiment, an assay that uses one hair follicle may be incubated for approximately 16 hours. In another embodiment, an assay that uses two hair follicles may be incubated for approximately four hours.

After sample incubation, the reaction may be stopped by addition of about 1/10^{th} volume of approximately 0.25 M Tris-HCl buffer, pH 8.7, and mixed. The pH may vary, in one embodiment between 8.5 to 9.0. Especially if the assay is performed or sold as part of a kit, the basic buffer may be provided as a separate container for pouring into the assay reaction container. In another embodiment, the basic buffer may be provided in a preloaded syringe, to be injected into the main reaction container at the appropriate time, either by manually pushing a plunger, or by some automatic or computerized control.

The absorbance at about 405 nm may then be read with a spectrophotometer or compared to a reference color card with a range of intensities corresponding to minoxidil sulfotransferase activity. Patients with a relatively high level of sulfotransferase activity will have a relatively strong colorimetric readout, resulting in a relatively significant color change. In comparison, patients with a relatively low level of sulfotransferase activity will have a relatively weak colorimetric readout, and correspondingly a relatively minimal color change. Patients with a strong colorimetric assay response would be expected to respond to minoxidil for hair re- growth or retention. Whereas, patients with a weak colorimetric assay response would be expected to have a poor response to minoxidil.

In yet another embodiment, the result from a patient's hair follicle colorimetric assay is used to determine an optimal treatment regime. Including, determining the method of delivery of the minoxidil (oral versus topical), or modifying the concentration and/or frequency of minoxidil therapy to suit the patient's minoxidil sulfotransferase activity. Furthermore, if a patient is unlikely to respond to minoxidil, finasteride may be recommended as an alternative to minoxidil.

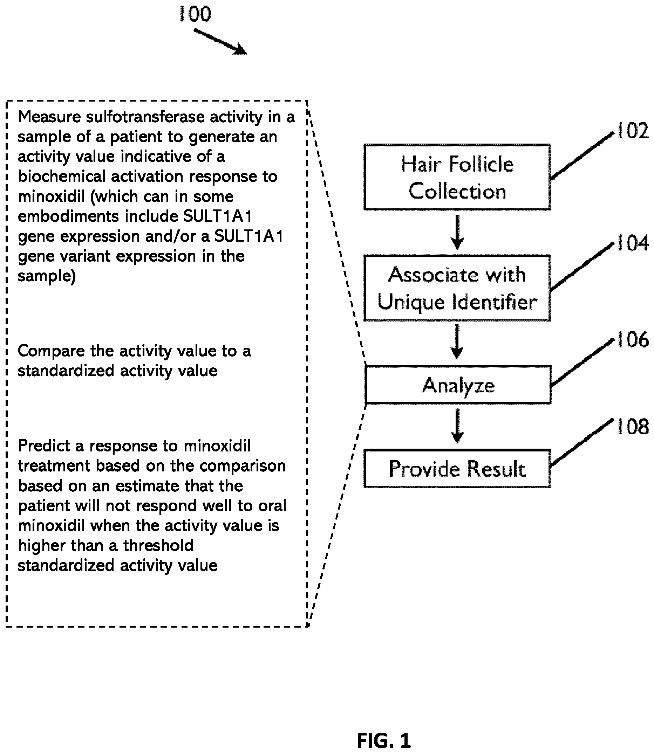

With reference to FIG. 1, a method 100 as described herein includes, at 102, collection of a hair follicle sample from a subject. The collection may be taken prior to and/or after beginning of minoxidil therapy. Then, at 104, the hair follicle sample may be coded with a unique identifier, for instance to protect privacy and facilitate handling. At 106, the hair follicle sample may be analyzed as described above. The analysis can be performed using the colorimetric assay described herein. The results of the analysis may then be provided to the subject or to the caregiver of the subject, at 108. The results of the analysis, each associated with its unique identifier, can be transmitted to a computer system that may include a Web-based server that is accessible, with proper authentication for instance using the unique identifier, by the subject or caregiver. The result, in addition to providing an indication of the likelihood that the patient will respond to 2%, 5% or greater minoxidil for the treatment of androgenetic alopecia, may also include a prediction of the dosage required and daily frequency of treatment by comparing a patient's minoxidil sulfotransferase activity level to a reference database. The result will also provide an indication of the likelihood that the patient will respond to oral or topical application of minoxidil for the treatment of androgenetic alopecia.

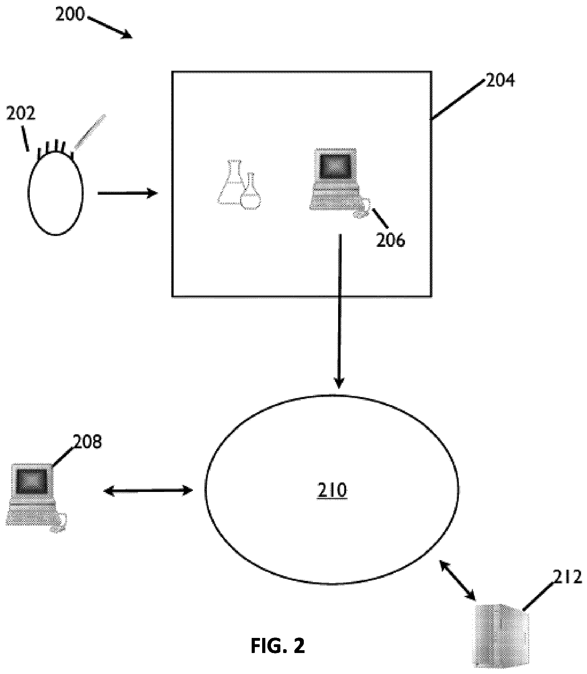

FIG. 2 schematically shows a system 200 for implementing the above procedure. A sample 202 of hair follicle from a subject is sent to a lab 204. An analysis of the sample in accordance with one or more of the afore-mentioned procedures is then conducted. Results of the analysis are for example compared with a database to generate an indication of the likelihood that the patient will respond to oral or topical application of minoxidil for the treatment of androgenetic alopecia. The database may be dynamic in nature, continuously updated for statistical adaptation based on past minoxidil treatment and response thereto, so that the database can adapt, or learn, from the patient pool and treatments over time, and in this manner become a better predictor of the likelihood of responders to the drug treatment. The database, or other entity or circuit or module capable of the adaptive scheme herein described, may reside in computer system 206 or separately therefrom. The outcome of the comparison and analysis can be forwarded to the subject's or caregiver's computer system 208, for example electronically by way of a network, such as the Internet, 210. Alternatively or in addition, the outcome of the comparison and analysis can be stored on a server 212 for accessing remotely by the subject or caregiver following proper authentication that may require reference to the unique identifier to preserve privacy.

The below Table 1 shows the result of testing for patient responsiveness to minoxidil. In one embodiment, the test is conducted by placing a hair follicle in an assay. The hair follicle may be collected prior to minoxidil therapy, or after start of minoxidil therapy. The follicle is placed in a solution containing minoxidil. The assay is then conducted as described above, and is examined to determine whether there is a SULT1A1 response. The results are then used as a predictor for patients who will respond well to minoxidil, and who will respond better to oral minoxidil versus topical application of minoxidil. Table 1 shows response raw-data values to the described testing. The first column is optical density resulting from the colorimetric testing. The second column indicates response. The testing indicates that methods used to predict oral (tablet) responses cannot be used to predict response to topical minoxidil.

The testing indicates that low sulfotransferase activity is significantly associated with response to topical minoxidil. Therefore, low SULT1A1 expression is a predictor that the patient will respond well to topical minoxidil. Conversely, high sulfotransferase activity is associated with a low likelihood to respond to oral minoxidil for androgenetic alopecia. Therefore, high SULT1A1 expression is a predictor that the patient will not respond well to oral minoxidil.

**Table 1**

| Optical Density (OD) | Response |
|---|---|
| 1.656 | 0 |
| 1.465 | 1 |
| 1.416 | 0 |
| 0.891 | 1 |
| 0.871 | 0 |
| 0.769 | 1 |
| 0.695 | 0 |
| 0.604 | 0 |
| 0.599 | 0 |
| 0.574 | 1 |
| 0.518 | 1 |
| 0.449 | 1 |
| 0.443 | 0 |
| 0.411 | 1 |
| 0.373 | 0 |
| 0.371 | 0 |
| 0.356 | 1 |
| 0.35 | 1 |
| 0.344 | 1 |
| 0.253 | 1 |
| 0.187 | 0 |
| 0.179 | 1 |
| 0.178 | 1 |
| 0.149 | 1 |
| 0.11 | 1 |
| 0.07 | 0 |

The testing indicates that there is a correlation between high SULT1A1 expression occurring in the hair follicle when exposed to minoxidil and a low likelihood of having good response to oral minoxidil. It is believed that this occurs as, when using the oral form, suffocation occurs in the liver and minoxidil is dissolved in tissue.

Additionally, or alternatively, the test can be used to examine genetic variations of the SULT1A1 gene, whereby variants that predispose to low SULT1A1 expression can be used to predict response to the route of minoxidil treatment, the dose of minoxidil treatment, and frequency of treatment, or a combination of the same.

It may also be possible to use a neural network to implement the system and method, to predict the likelihood that the patient will respond to minoxidil for the treatment of androgenetic alopecia based on the patient's minoxidil sulfotransferase activity profile. According to such an approach, for predicting the likelihood of response to the drug treatment can include (a) constructing an N-layer neural network, and (b) training the neural network with a data set of patients' outcomes to treatment with minoxidil for androgenetic alopecia along with the patients' minoxidil sulfotransferase activity profiles, (c) obtaining a hair follicle sample from the subject (d) generating a minoxidil sulfotransferase activity profile from the sample, the profile being a function of values associated with a prescribed set of minoxidil sulfotransferase activity levels; (e) inputting the subjects minoxidil sulfotransferase activity profile into the neural network; (f) obtaining a value or set of values from the neural network indicative of the patient's expected outcome (respondent) to the drug treatment at a single or multiple dosages; and (g) providing the patient the drug treatment at the recommended dosage.

As can be appreciated from the present disclosure, embodiments can relate to a method for predicting response to minoxidil. The method can involve measuring sulfotransferase activity in a sample of a patient to generate an activity value indicative of a biochemical activation response to minoxidil. Measuring sulfotransferase activity, in some cases, can include measuring SULT1A1 gene expression and/or a SULT1A1 gene variant expression in the sample. Measuring sulfotransferase activity in the sample of a patient can involve obtaining one or more hair follicles and/or one or more scalp biopsies as the sample. For instance, the sample may be subjected to a colorimetric assay to determine the level of sulfotransferase activity. For instance, measuring sulfotransferase activity in the sample of the patient can involve obtaining one or more hair follicles and/or one or more scalp biopsies as the sample, and measuring sulfotransferase activity in the sample of the patient is performed via an assay device, wherein the method involves:
i. placing the one or more hair follicles or one or more scalp biopsies in a reaction mixture containing potassium phosphate buffer, magnesium chloride, adenosine 3',5'-diphosphate (PAP) or adenosine 3'-phosphate ,5'-phosphosulfate (PAPS), p-nitrophenyl sulfate, and minoxidil;
ii. mixing the reaction mixture; incubating the reaction mixture for a predetermined length; and
iii. stopping the reaction.

As a non-limiting example, a patient's hair follicle sample (from 1 to n hair follicles) may be placed in a reaction mixture containing about 50 mM potassium phosphate buffer (pH approximately 6.5), about 5 mM magnesium chloride, about 20 µM adenosine 3',5'-diphosphate (PAP) or adenosine 3'-phosphate,5'-phosphosulfate (PAPS), about 5 mM p-nitrophenyl sulfate and about 0.1 mM minoxidil. This reaction may take place in a transparent container with a lid or other opening in which the hair follicle samples may be inserted.

As part of the above reaction, it is understood that in the presence of minoxidil sulfotransferase activity, p-nitrophenyl sulfate is converted to the colorimetric p-nitrophenolate. Absorbance at about 405 nm may then be read with a spectrophotometer or compared to a reference color card with a range of intensities corresponding to minoxidil sulfotransferase activity. Patients with a relatively high level of sulfotransferase activity will have a relatively strong colorimetric readout, resulting in a relatively significant color change. In comparison, patients with a relatively low level of sulfotransferase activity will have a relatively weak colorimetric readout, and correspondingly a relatively minimal color change. The intensity of the colorimetric readout can be quantified - e.g., a very weak readout (or weak intensity) can be assigned an activity value of zero and a very strong readout (or strong intensity) can be assigned 18ctivetivity value of 1. Gradations of the readout can be assigned values between 0 and 1. Use of a 0 to 1 scale is for exemplary purposes.

It should be noted that measuring sulfotransferase activity in the sample of a patient can occur before (e.g., 1-14 days prior), during, and/or after the patient is being administered a minoxidil treatment. Thus, the methods disclosed herein are not limited to measuring sulfotransferase activity during or after minoxidil treatment.

The method can involve comparing the activity value to a standardized activity value. Standardized activity values can be determined based on empirical data obtained from a plurality of subjects who had undergone minoxidil treatment and their responses measured. The comparison can be done by comparing the activity value to a lookup table containing plural standardized activity values, for example. A prediction of a response to minoxidil treatment can be made based on the comparison.

In some embodiments, the minoxidil treatment can be treatment for androgenetic alopecia. The response to minoxidil treatment can be increased or improved hair growth or hair retention. Predicting a response to minoxidil treatment can involve estimating a response based on a minoxidil dosage amount, a minoxidil dosage frequency, and/or a minoxidil dosage routine.

As noted herein, the minoxidil treatment can be oral minoxidil or topical minoxidil. Also noted herein is that low SULT1A1 gene expression and/or a SULT1A1 gene variant expression (or low activity) can be a predictor that a patient will respond well to topical minoxidil treatment. High SULT1A1 gene expression and/or a SULT1A1 gene variant expression (or high activity) can be a predictor that a patient will not respond well to oral minoxidil treatment. Thus, predicting a response to minoxidil treatment can involve estimating that the patient will respond well to topical minoxidil when the activity value is lower than a threshold standardized activity value, and estimating that the patient will not respond well to oral minoxidil when the activity value is higher than a threshold standardized activity value. The threshold values will depend on the empirical data collected, the class (e.g., male, female, race, type of alopecia, etc.) of individual, how the data is trained (if training of data is performed), etc. It is understood that the standardized activity value can pertain to biochemical activation response to minoxidil associated with hair growth or hair retention for one or more class of individuals. In some cases, this can pertain to gene expression and/or SULT1A1 gene variant expression associated with hair growth or hair retention for one or more class of individuals. Thus, the comparison can take into account the class of individuals the patient from which the sample is taken falls within. The patient may fall within one or more classes of individuals. Some embodiments can involve generating plural activity values indicative of the biochemical activation response to minoxidil (which can in some embodiment be indicative of gene expression and/or a SULT1A1 gene variant expression in the sample), and comparing the plural activity values to plural standardized activity values. In an exemplary embodiment, the standardized activity value can pertain to biochemical activation response to minoxidil associated with hair growth or hair retention at which a statistical sample of subjects show high or low minoxidil response for a minoxidil dosage amount, a minoxidil dosage frequency, and/or a minoxidil dosage routine.

In some embodiments, genetic testing of SLC family genes (e.g., SLC22A9 gene) can be used to predict a response to minoxidil treatment. The genetic testing of one or more genes in the SLC family can generate results that predict low SLC activity or expression. For instance, a prediction of low activity or expression of one or more genes in the SLC family for a patient can be an indicator that there is or will be a low oral minoxidil response. A prediction of high activity or expression of one or more genes in the SLC family for a patient can be an indicator that there is or will be a high oral minoxidil response.

In some embodiments, genetic testing of AABC family genes (e.g., AABC3 gene) can used to predict a response to minoxidil treatment. The genetic testing of one or more genes in the AABC family can generate results that predict high AABC activity or expression. For instance, a prediction of high activity or expression of one or more genes in the AABC family for a patient can be an indicator that there is or will be a low oral minoxidil response. A prediction of low activity or expression of one or more genes in the AABC family for a patient can be an indicator that there is or will be a high oral minoxidil response.

It is understood that activity or expression of a gene or a genetic can includes activity or expression of a protein, a mRNA, a DNA, etc.

In some embodiments, blood flow in the skin (e.g., the scalp) of a patient can be detected via Doppler velocitometry (LDV), photopulse plethysmography, near infrared imaging, thermal imaging, optical coherence tomography, orthogonal polarization spectral imaging, fluorescence imaging, laser Doppler imaging, microscopy, spatial frequency domain imaging, photoacoustic detection, spectral imaging, hyperspectral imaging, and electromagnetic induction. Surrogate measurement(s) such as tissue oxygenation can be used to measure the blood flow as well. Blood flow in the skin can be used as an indicator to predict the efficacy of minoxidil for the treatment of alopecia. Details of the relation between blood flow and efficacy of minoxidil can be appreciated from International Application Publication No. WO2017007921, which is incorporated herein by reference in its entirety. With embodiments using Doppler techniques, a lower Doppler velocity can be an indicator that there is a high oral minoxidil response and/or a low topical response to minoxidil.

### Examples

### Data Overview

A total of 123 subjects recruited. Overall among subjects with OD < 0.37 85% responded to oral minoxidil (5mg men and 1.25mg women). Overall among subjects with OD > 0.37 43% responded to oral minoxidil (5mg men and 1.25mg women). This was statistically significant p=0.0088 (Fischer Exact Test). In this cohort, oral minoxidil only worked on 42% of women and 73% of men.

### Discussion

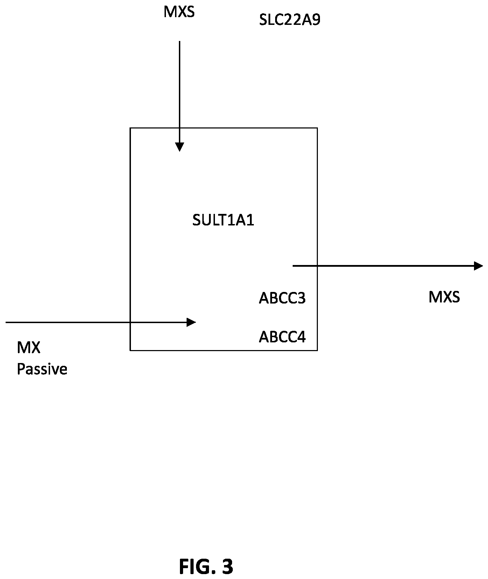

The study supports the use of an assay (e.g, a Mediator Release Test (MRT) assay) to distinguish responder from non-responder to oral minoxidil; however, it was discovered that oral minoxidil works the opposite to topical minoxidil for treatment of androgenetic alopec-a - i.e., lower SULT1A1 enzymatic activity in the hair follicle predicts oral minoxidil response. This is a significant and unexpected discovery since this implies that oral minoxidil is sulfonated in the liver, but the response is determined by the influx transporter SLC22A9 in the hair follicle. The low or high activity of SULT1A1 in the hair follicle is an indirect marker for the transport expression. Patients with high SULT1A1 expression in hair follicles have a corresponding high ABCC3 efflux transporter expression and low SLC22A9 influx transporter expression; thus, resulting in low response to oral minoxidil. The converse if also true of patients with low SULT1A1 expression in hair follicles (see FIG. 3).

Classification models/techniques, artificial intelligence, etc. can be used to predict the response to minoxidil treatment. For instance, the empirical data for the standardized activity values can be a trained data set stored in a data store (e.g., database). A classification model can be implemented on a processor (e.g., a computer device) to compare the activity value(s) to the standardized activity value(s) and/or make predictions or estimates about minoxidil treatment response. In developing the standardized data set, activity data can be obtained from one or more individuals to generate a robust data set can allow the model to be used to determine trends, predict response states, use multivariate analyses regarding conditions and factors (e.g., race, age, gender, type of alopecia, minoxidil dosage amount, minoxidil dosage frequency, minoxidil dosage routine, etc.) that cause or relate to a response, etc. For instance, multivariable modeling techniques can be used to determine which conditions or factors statistically contribute to a response, a change in response, etc. The multivariable modeling technique can include one or more of logistic regression with or without cubic splines, random forest, xgboost, support vector machines, nearest neighbor, artificial neural networks, and/or long short-term memory (LSTM), multivariate analysis of variance (MANOVA), multivariate analysis of covariance (MANCOVA), principal components analysis (PCA), canonical correlation analysis, redundancy analysis (RDA), correspondence analysis (CA), canonical correspondence analysis (CCA), multidimensional scaling, discriminant analysis, linear discriminant analysis (LDA), clustering systems, recursive adaptive partitioning, vector autoregression, principal response curves analysis (PRC), etc. In an exemplary embodiment, predicting the response to minoxidil treatment can involve implementation of a neural network.

As can be appreciated, implementation of the methods disclosed herein be via a computer device or a processor. For instance, a computer device including a processor and memory can be in communication with the assay device (which can also have a processor and memory). Each of these devices can have transceivers, transmitters, communication bus, etc. to facilitate communication (wireless or hardwired) between the two.

Any of the processors disclosed herein can be part of or in communication with a machine (e.g., a computer device, a logic device, a circuit, an operating module (hardware, software, and/or firmware), etc.). The processor can be hardware (e.g., processor, integrated circuit, central processing unit, microprocessor, core processor, computer device, etc.), firmware, software, etc. configured to perform operations by execution of instructions embodied in computer program code, algorithms, program logic, control, logic, data processing program logic, artificial intelligence programming, machine learning programming, artificial neural network programming, automated reasoning programming, etc.

Any of the processors disclosed herein can be a scalable processor, a parallelizable processor, a multi-thread processing processor, etc. The processor can be a computer in which the processing power is selected as a function of anticipated network traffic (e.g., data flow). The processor can include any integrated circuit or other electronic device (or collection of devices) capable of performing an operation on at least one instruction, which can include a Reduced Instruction Set Core (RISC) processor, a Complex Instruction Set Computer (CISC) microprocessor, a Microcontroller Unit (MCU), a CISC-based Central Processing Unit (CPU), a Digital Signal Processor (DSP), a Graphics Processing Unit (GPU), a Field Programmable Gate Array (FPGA), etc. The hardware of such devices may be integrated onto a single substrate (e.g., silicon "ie"), or distributed among two or more substrates. Various functional aspects of the processor may be implemented solely as software or firmware associated with the processor.

The processor can include one or more processing or operating modules. A processing or operating module can be a software or firmware operating module configured to implement any of the functions disclosed herein. The processing or operating module can be embodied as software and stored in memory, the memory being operatively associated with the processor. A processing module can be embodied as a web application, a desktop application, a console application, etc.

The processor can include or be associated with a computer or machine readable medium. The computer or machine readable medium can include memory. Any of the memory discussed herein can be computer readable memory configured to store data. The memory can include a volatile or non-volatile, transitory or non-transitory memory, and be embodied as an in-memory, an active memory, a cloud memory, etc. Examples of memory can include flash memory, Random Access Memory (RAM), Read Only Memory (ROM), Programmable Read only Memory (PROM), Erasable Programmable Read only Memory (EPROM), Electronically Erasable Programmable Read only Memory (EEPROM), FLASH-EPROM, Compact Disc (CD)-ROM, Digital Optical Disc DVD), optical storage, optical medium, a carrier wave, magnetic cassettes, magnetic tape, magnetic disk storage or other magnetic storage devices, or any other medium which can be used to store the desired information and which can accessed by the processor.

The memory can be a non-transitory computer-readable medium. The term "computer-readable medium" (or "machine-readable medium") as used herein is an extensible term that refers to any medium or any memory, that participates in providing instructions to the processor for execution, or any mechanism for storing or transmitting information in a form readable by a machine (e.g., a computer). Such a medium may store computer-executable instructions to be executed by a processing element and/or control logic, and data which is manipulated by a processing element and/or control logic, and may take many forms, including but not limited to, non-volatile medium, volatile medium, transmission media, etc. The computer or machine readable medium can be configured to store one or more instructions thereon. The instructions can be in the form of algorithms, program logic, etc. that cause the processor to execute any of the functions disclosed herein.

Embodiments of the memory can include a processor module and other circuitry to allow for the transfer of data to and from the memory, which can include to and from other components of a communication system. This transfer can be via hardwire or wireless transmission. The communication system can include transceivers, which can be used in combination with switches, receivers, transmitters, routers, gateways, wave-guides, etc. to facilitate communications via a communication approach or protocol for controlled and coordinated signal transmission and processing to any other component or combination of components of the communication system. The transmission can be via a communication link. The communication link can be electronic-based, optical-based, opto-electronic-based, quantum-based, etc. Communications can be via Bluetooth, near field communications, cellular communications, telemetry communications, Internet communications, etc.

Transmission of data and signals can be via transmission media. Transmission media can include coaxial cables, copper wire, fiber optics, etc. Transmission media can also take the form of acoustic or light waves, such as those generated during radio-wave and infrared data communications, or other form of propagated signals (e.g., carrier waves, digital signals, etc.).

Any of the processors can be in communication with other processors of other devices (e.g., a computer device, a computer system, a laptop computer, a desktop computer, etc.). Any of the processors can have transceivers or other communication devices / circuitry to facilitate transmission and reception of wireless signals. Any of the processors can include an Application Programming Interface (API) as a software intermediary that allows two or more applications to talk to each other.

Another exemplary embodiment can relate to a method for selecting a treatment for a human subject suffering from androgenetic alopecia. The method can involve measuring sulfotransferase activity in a sample of a patient to generate an activity value indicative of a biochemical activation response to minoxidil (which in some embodiments can include SULT1A1 gene expression and/or a SULT1A1 gene variant expression in the sample). The method can involve comparing the activity value to a standardized activity value. The method can involve predicting a response to minoxidil treatment based on the comparison. The method can involve prescribing a minoxidil dosage amount, a minoxidil dosage frequency, and/or a minoxidil dosage routine as the androgenetic alopecia treatment.

In some embodiments, prescribing the minoxidil dosage amount, the minoxidil dosage frequency, and/or the minoxidil dosage routine can involve prescribing oral minoxidil or topical minoxidil.

In some embodiments, predicting a response to minoxidil treatment can involve estimating that the patient will respond well to topical minoxidil when the activity value is lower than a threshold standardized activity value, and estimating that the patient will not respond well to oral minoxidil when the activity value is higher than a threshold standardized activity value.

In some embodiments, prescribing the androgenetic alopecia treatment can involve prescribing topical minoxidil when the activity value is lower than the threshold standardized activity value, and not prescribing oral minoxidil when the activity value is higher than the threshold standardized activity value.

The above are exemplary modes of carrying out the invention and are not intended to be limiting. It will be apparent to those of ordinary skill in the art that modifications thereto can be made without departure from the spirit and scope of the invention as set forth in the following claims.

## Claims

1. A method for predicting response to minoxidil, the method comprising:
measuring gene, genetic, and/or enzymatic activity in a sample of a patient to generate an activity value indicative of a biochemical activation response to minoxidil;
comparing the activity value to a standardized activity value; and
predicting a response to minoxidil treatment based on the comparison.

2. The method of claim 1, wherein:
measuring gene, genetic, and/or enzymatic activity includes:
measuring SULT1A1 gene expression, a SULT1A1 gene variant expression, and/or SULT1A1 enzymatic activity in the sample ;
measuring SLC22A9 gene expression, SCL22A9 gene variant expression, SLC22A9 transporter activity from a hair cell in the sample, and/or measuring SLC22A9 transporter activity;
measuring AABC3 gene expression and/or AABC3 gene variant expression in the sample; and/or
measuring blood flow in the patient
preferably, wherein:
predicting a response to minoxidil treatment involves:
when the gene, genetic, and/or enzymatic activity relates to SULT1A1:
estimating that the patient will respond to oral minoxidil when the activity value is lower than a threshold standardized activity value; and
estimating that the patient will not respond well to oral minoxidil when the activity
value is higher than a threshold standardized activity value;
when the gene, genetic, and/or SLC22A9 transporter activity from the hair cell relates to SLC22A9:
estimating that the patient will respond to oral minoxidil when the activity value is higher than a threshold standardized activity value; and
estimating that the patient will not respond well to oral minoxidil when the activity value is lower than a threshold standardized activity value; and
when the gene and/or genetic activity relates to AABC3:
estimating that the patient will respond to oral minoxidil when the activity value is lower than a threshold standardized activity value; and
estimating that the patient will not respond well to oral minoxidil when the activity value is higher than a threshold standardized activity value.

3. The method of claim 1, wherein:
the minoxidil treatment is oral minoxidil.

4. The method of claim 1, wherein:
measuring blood flow of the patient involves Doppler velocitometry;
predicting a response to minoxidil treatment involves:
estimating that the patient will respond well to oral minoxidil when the Doppler velocity is lower than a threshold value; and
estimating that the patient will not respond well to oral minoxidil when the Doppler velocity is higher than the threshold value,
and/or wherein
measuring SLC22A9 transport activity from the hair cell involves use of an uptake assay,
and/or wherein
when the gene, genetic, and/or enzymatic activity relates to SULT1A1:
estimating that the patient will respond well to oral minoxidil when the activity value is lower than a threshold standardized activity value that is within a range from 0.37 to 0.4; and
estimating that the patient will not respond well to oral minoxidil when the activity value is higher than a threshold standardized activity value that is within a range from 0.37 to 0.4,
and/or wherein:
the minoxidil treatment is treatment for androgenetic alopecia,

5. The method of claim 1, comprising:
generating plural activity values indicative of the biochemical activation response to minoxidil; and
comparing the plural activity values to plural standardized activity values.

6. The method of claim 1, wherein:
the response to minoxidil treatment is increased or improved hair growth or hair retention,
and/or wherein:
predicting a response to minoxidil treatment involves estimating the biochemical activation response based on a minoxidil dosage amount, a minoxidil dosage frequency, and/or a minoxidil dosage routine,
and/or wherein:
the standardized activity value pertains to biochemical activation response to minoxidil associated with hair growth or hair retention for a class of individuals,
and/or wherein:
the standardized activity value pertains to biochemical activation response to minoxidil associated with hair growth or hair retention at which a statistical sample of subjects show high or low minoxidil response for a minoxidil dosage amount, a minoxidil dosage frequency, and/or a minoxidil dosage routine,
and/or wherein:
measuring gene, genetic, and/or enzymatic activity in the sample of a patient involves obtaining one or more hair follicles and/or one or more scalp biopsies as the sample,
and/or wherein:
measuring gene, genetic, and/or enzymatic activity in the sample of a patient occurs before, during, and/or after the patient is being administered a minoxidil treatment, and/ or wherein:
measuring sulfotransferase activity in the sample of a patient occurs 1 to 14 days prior to the patient being administered a minoxidil treatment,
and/or wherein:
measuring gene, genetic, and/or enzymatic activity in the sample of a patient is performed via an assay device,
and/or wherein:
measuring gene, genetic, and/or enzymatic activity in the sample of a patient is performed via a colorimetric assay device.

7. The method of claim 1, wherein:
predicting the response to minoxidil treatment involves implementation of a neural network.

8. The method of claim 1, wherein measuring gene, genetic, and/or enzymatic activity in the sample of the patient involves obtaining one or more hair follicles and/or one or more scalp biopsies as the sample, and measuring gene, genetic, and/or enzymatic activity in the sample of the patient is performed via an assay device, the method comprising:
placing the one or more hair follicles or one or more scalp biopsies in a reaction mixture containing potassium phosphate buffer, magnesium chloride, adenosine 3',5'-diphosphate (PAP) or adenosine 3'-phosphate,5'-phosphosulfate (PAPS), p-nitrophenyl sulfate, and minoxidil;
mixing the reaction mixture;
incubating the reaction mixture for a predetermined length; and
stopping the reaction.

9. A method for selecting a treatment for a human subject suffering from androgenetic alopecia, the method comprising:
measuring gene, genetic activity, and/or enzymatic in a sample of a patient to generate an activity value indicative a biochemical activation response to minoxidil;
comparing the activity value to a standardized activity value;
predicting a response to minoxidil treatment based on the comparison; and
prescribing a minoxidil dosage amount, a minoxidil dosage frequency, and/or a minoxidil dosage routine as the androgenetic alopecia treatment.

10. The method of claim 9, wherein:
measuring gene, genetic, and/or enzymatic activity includes:
measuring SULT1A1 gene expression, SULT1A1 gene variant expression, and/or SULT1A1 enzymatic activity in the sample;
measuring SLC22A9 gene expression, SCL22A9 gene variant expression SLC22A9 transporter activity from a hair cell in the sample, and/or SCL22A9 transporter activity;
measuring AABC3 gene expression and/or AABC3 gene variant expression in the sample; and/or
measuring blood flow in the patient.

11. The method of claim 9, wherein:
prescribing the minoxidil dosage amount, the minoxidil dosage frequency, and/or the minoxidil dosage routine involves prescribing oral minoxidil.

12. The method of claim 9, wherein.
measuring blood flow of the patient involves Doppler velocitometry;
predicting a response to minoxidil treatment involves:
estimating that the patient will respond well to oral minoxidil when the Doppler velocity is lower than a threshold value; and
estimating that the patient will not respond well to oral minoxidil when the Doppler velocity is higher than the threshold value.

13. The method of claim 9, wherein:
predicting a response to minoxidil treatment involves:
when the gene, genetic, and/or enzymatic activity relates to SULT1A1:
estimating that the patient will respond to oral minoxidil when the activity value is lower than a threshold standardized activity value; and
estimating that the patient will not respond well to oral minoxidil when the activity value is higher than a threshold standardized activity value;
when the gene, genetic and/or SLC22A9 transporter activity from the hair cell relates to SLC22A9:
estimating that the patient will respond to oral minoxidil when the activity value is higher than a threshold standardized activity value; and
estimating that the patient will not respond well to oral minoxidil when the activity value is lower than a threshold standardized activity value; and
when the gene and/or genetic activity relates to AABC3:
estimating that the patient will respond to oral minoxidil when the activity value is lower than a threshold standardized activity value and estimating that the patient will not respond well to oral minoxidil when the activity value is higher than a threshold standardized activity value.

14. The method of claim 9, wherein when the gene, genetic and/or enzymatic activity relates to SULT1A1:
estimating that the patient will respond well to oral minoxidil when the activity value is lower than a threshold standardized activity value that is within a range from 0.37 to 0.4; and estimating that the patient will not respond well to oral minoxidil when the activity value is higher than a threshold standardized activity value that is within a range from 0.37 to 0.4.

15. The method of claim 9, wherein measuring SLC22A9 transport activity from the hair cell involves use of an uptake assay.
